(19) Europäisches Patentamt
European Patent Office
Office européen des brevets

(11) Veröffentlichungsnummer: **0 003 584**
**B1**

(12) # EUROPÄISCHE PATENTSCHRIFT

(21) Anmeldenummer: **79100330.4**

(22) Anmeldetag: **05.02.79**

(51) Int. Cl.³: **C 07 C 59/58,**
A 01 N 37/38, C 07 C 69/67

(54) **Phenoxypropionsäure-Derivate, Verfahren zu ihrer Herstellung und ihre Verwendung als Herbizide**

(30) Priorität: **13.02.78 DE 2805981**

(43) Veröffentlichungstag der Anmeldung:
**22.08.79 Patentblatt 79/17**

(45) Bekanntmachung des Hinweises auf die Patenterteilung:
**21.01.81 Patentblatt 81/03**

(84) Benannte Vertragsstaaten:
**BE CH DE FR GB IT NL**

(56) Entgegenhaltungen:
**DE - A - 2 637 098**
**DE - A - 2 643 438**
**DE - A - 2 716 189**
**DE - A - 2 732 442**
**DE - A - 2 809 541**

(73) Patentinhaber: **BAYER AG**
**Zentralbereich Patente, Marken und Lizenzen**
**D - 5090 Leverkusen 1**
**Bayerwerk (DE)**

(72) Erfinder: **Förster, Heinz, Dr.**
**Am Eckbusch 47**
**D - 5600 Wuppertal 1 (DE)**
**Eue, Ludwig, Dr**
**Paul-Klee-Strasse 36**
**D - 5090 Leverkusen 1 (DE)**
**Schmidt, Robert Rudolf, Dr.**
**Hahnenweg 5**
**D - 5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 584 B1

Phenoxypropionsäure-Derivate, Verfahren zu ihrer Herstellung und ihre Verwendung als Herbizide

Die vorliegende Erfindung betrifft neue Phenoxy-propion-säure-Derivate, mehrere Verfahren zu ihrer Herstellung sowie ihre Verwendung als Herbizide.

Es ist bereits bekannt geworden daß 3-(4-Chlorphenoxy)-$\alpha$-phenoxy-propionsäure-äthylester zur Bekämpfung von Unkraut geeignet ist (vgl. DT—OS 716 189). Die Wirksamkeit dieses Stoffes ist jedoch, insbesondere bei niedrigen Dosierungen, nicht immer ganz befriedigend.

Gegenstand dieser Erfindung sind neue Phenoxypropionsäure-Derivate der Formel

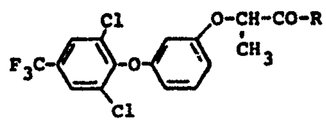
(I)

in welcher

R für Hydroxy, gegebenenfalls durch Alkoxy, Alkylthio, Carbalkoxy, Phenylmethylaminocarbonyl und/oder Halogen substituiertes Alkoxy, Amino, Hydrazino, gegebenenfalls durch Alkoxy oder Dialkylamino substituiertes Alkylamino, Dialkylamino, Alkenylamino, Arylamino oder N-Aryl-N-alkylamino, wobei der Arylrest jeweils durch Alkyl, Alkoxy, Carbalkoxy, Halogen und/oder Nitro substituiert sein kann, ferner für einen gegebenenfalls durch Alkyl substituierten, über Stickstoff gebundenen gesättigten heterocyclischen Ring mit 5 oder 6 Ringgliedern, oder für den Rest der Formel

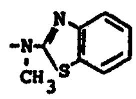

steht.

Gegenstand der Erfindung sind auch Verfahren zur Herstellung der Phenoxypropionsäure-Derivate der Formel (I). So erhält man

a) die Verbindungen der Formel (I), indem man das Phenoxypropionsäurechlorid der Formel

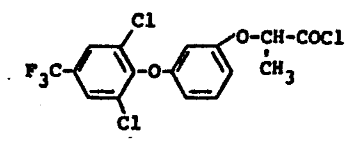
(II)

mit Verbindungen der Formel

HR (III)

in welcher

R die oben angegebene Bedeutung hat,

gegebenenfalls in Gegenwart eines Säureakzeptors sowie gegebenenfalls in Gegenwart eines Verdünnungsmittels umsetzt,

oder

b) diejenige Verbindung der Formel (I), in der R für Methoxy steht, indem man den Diphenyläther der Formel

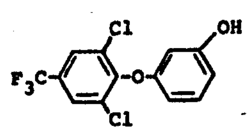
(IV)

mit $\alpha$-Brompropionsäure-methylester der Formel

$$Br—\underset{\displaystyle CH_3}{\underset{|}{CH}}—COOCH_3 \quad (V)$$

in Gegenwart eines Säureakzeptors sowie in Gegenwart eines Verdünnungsmittels umsetzt.

und

c) diejenige Verbindung der Formel (I), in der R für Hydroxyl steht, indem man die Verbindung der Formel

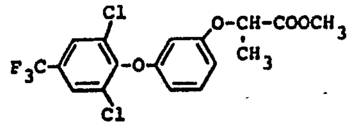

(I')

mit Basen in Gegenwart eines Verdünnungsmittels behandelt und anschließend ansäuert, oder

d) diejenigen Verbindungen der Formel (I), in denen R für Alkoxy mit mehr als einem Kohlenstoffatom steht, indem man die Verbindung der Formel

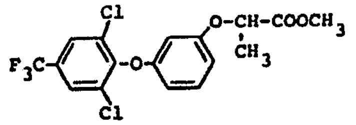

(I')

mit Alkoholen der Formel

$HOR^1$ (VI)

in welcher

$R^1$ für Alkyl mit mehr als einem Kohlenstoffatom steht, gegebenenfalls in Gegenwart eines Alkoholates sowie gegebenenfalls in Gegenwart eines Verdünnungsmittels umsetzt.

Gegenstand dieser Erfindung ist ferner die Verwendung von Phenoxypropionsäure-Derivaten der Formel (I) als Herbizide.

Überraschenderweise besitzen die erfindungsgemäßen Phenoxypropionsäure-Derivate sehr gute herbizide Eigenschaften. So zeichnen sich die erfindungsgemäßen Stoffe durch eine hervorragende Wirksamkeit gegen Cyperus aus. Darüber hinaus sind sie zur Bekämpfung von Unkräutern in Getreide, Mais und Reis geeignet. Die erfindungsgemäßen Stoffe stellen somit eine wertvolle Bereicherung der Technik dar.

Die erfindungsgemäßen Phenoxypropionsäure-Derivate sind durch die Formel (I) allgemein definiert. In der Formel (I) steht R vorzugsweise für Hydroxyl oder geradkettiges oder verzweigtes Alkoxy mit 1 bis 4 Kohlenstoffatomen, wobei jeder dieser Alkoxyreste ein- oder mehrfach, gleichartig oder verschieden substituiert sein kann. Als Substituenten kommen hierbei vorzugsweise in Frage Alkoxy mit 1 bis 4, insbesondere 1 bis 2 Kohlenstoffatomen, Alkylthio mit 1 bis 4, insbesondere 1 bis 2 Kohlenstoffatomen, Carbalkoxy mit 1 bis 4, insbesondere 1 bis 2 Kohlenstoffatomen in der Alkoxygruppe, Phenylmethylaminocarbonyl sowie Chlor und Brom.

Weiterhin steht R vorzugsweise für Amino, Hydrazino, gegebenenfalls durch Alkoxy mit 1 bis 4 Kohlenstoffatomen oder durch Dialkylamino mit 1 bis 4 Kohlenstoffatomen je Alkylgruppe substituiertes Alkylamino mit 1 bis 4 Kohlenstoffatomen, Dialkylamino mit 1 bis 4 Kohlenstoffatomen je Alkylgruppe, Alkenylamino mit bis zu 4 Kohlenstoffatomen oder für Phenylamino bzw. N-Phenyl-N-alkyl-amino mit 1 bis 4 Kohlenstoffatomen in der Alkylgruppe, wobei der Phenylrest jeweils substituiert sein kann durch Alkyl mit 1 bis 4 Kohlenstoffatomen, Alkoxy mit 1 bis 4 Kohlenstoffatomen, Carbalkoxy mit 1 bis 4 Kohlenstoffatomen in der Alkoxygruppe, Fluor, Chlor, Brom und/oder Nitro. Darüber hinaus steht R vorzugsweise für einen gegebenenfalls durch Methyl substituierten, über Stickstoff gebundenen gesättigten heterocyclischen Ring mit 5 oder 6 Ringgliedern, wobei in dem Ring außer dem an die Carbonylgruppe gebundenen Stickstoffatom noch ein weiteres Stickstoff- oder Sauerstoffatom enthalten sein kann. Als Beispiele für derartige Heterocyclen seien genannt Piperidin, 2-Methyl-piperidin, Pyrrolidin, Piperazin, Pyrazolidin und Morpholin. Schließlich steht R vorzugsweise für den Rest der Formel

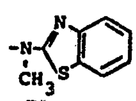

Als Beispiele für erfindungsgemäße Phenoxypropionsäure-Derivative der Formel (I) seien in einzelnen gennant:

3-(2,6-Dichlor-4-trifluormethyl-phenoxy)-$\alpha$-phenoxypropionsäure
3-(2,6-Dichlor-4-trifluormethyl-phenoxy)-$\alpha$-phenoxy-propionsäure-methylester
3-(2,6-Dichlor-4-trifluormethyl-phenoxy)-$\alpha$-phenoxy-propionsäure-äthylester
3-(2,6-Dichlor-4-trifluormethyl-phenoxy)-$\alpha$-phenoxy-propionsäure-isopropylester
3-(2,6-Dichlor-4-trifluormethyl-phenoxy)-$\alpha$-phenoxy-propionsäure-n-butylester
3-(2,6-Dichlor-4-trifluormethyl-phenoxy)-$\alpha$-phenoxy-propionsäure-(2-methoxy)-äthylester
3-(2,6-Dichlor-4-trifluormethyl-phenoxy)-$\alpha$-phenoxy-propionsäure-(2-methylthio)-äthylester
3-(2,6-Dichlor-4-trifluormethyl-phenoxy)-$\alpha$-phenoxy-propionsäure-(2-äthoxycarbonyl)-äthylester
3 - (2,6 - Dichlor - 4 - trifluormethyl - phenoxy) - $\alpha$ - phenoxy - propionsäure - (2 - phenylmethylaminocarbonyl) - äthylester

3-(2,6-Dichlor-4-trifluormethyl-phenoxy)-α-phenoxy-propionsäure-(2-chlor)-äthylester
3-(2,6-Dichlor-4-trifluormethyl-phenoxy)-α-phenoxy-propionsäure-methylamid
3-(2,6-Dichlor-4-trifluormethyl-phenoxy)-α-phenoxy-propionsäure-(2-methoxy)-äthylamid
3-(2,6-Dichlor-4-trifluormethyl-phenoxy)-α-phenoxy-propionsäure-(2-dimethylamino)-äthylamid
3-(2,6-Dichlor-4-trifluormethyl-phenoxy)-α-phenoxy-propionsäure-dimethylamid
3-(2,6-Dichlor-4-trifluormethyl-phenoxy)-α-phenoxy-propionsäure-buten-2-yl-amid
3-(2,6-Dichlor-4-trifluormethyl-phenoxy)-α-phenoxy-propionsäure-(4-methyl-phenyl)-amid
3-(2,6-Dichlor-4-trifluormethyl-phenoxy)-α-phenoxy-propionsäure-(4-methoxy-phenyl)-amid
3-(2,6-Dichlor-4-trifluormethyl-phenoxy)-α-phenoxy-propionsäure-(4-äthoxycarbonyl-phenyl)-amid
3-(2,6-Dichlor-4-trifluormethyl-phenoxy)-α-phenoxy-propionsäure-(4-chlor-phenyl)-amid
3-(2,6-Dichlor-4-trifluormethyl-phenoxy)-α-phenoxy-propionsäure-(4-nitro-phenyl)-methylamid
3-(2,6-Dichlor-4-trifluormethyl-phenoxy)-α-phenoxy-propionsäure-piperidid
3-(2,6-Dichlor-4-trifluormethyl-phenoxy)-α-phenoxy-propionsäure-pyrrolidid
3-(2,6-Dichlor-4-trifluormethyl-phenoxy)-α-phenoxy-propionsäure-piperazid
3-(2,6-Dichlor-4-trifluormethyl-phenoxy)-α-phenoxy-propionsäure-pyrazolidid
3-(2,6-Dichlor-4-trifluormethyl-phenoxy)-α-phenoxy-propionsäure-morpholid

Verwendet man 3-(2,6-Dichlor-4-trifluormethyl-phenoxy)-α-phenoxy-propionsäurechlorid und Diäthylamin als Ausgangsstoffe, so kann der Reaktionsablauf nach dem erfindungsgemäßen Verfahren (a) durch das folgende Formelschema wiedergegeben werden:

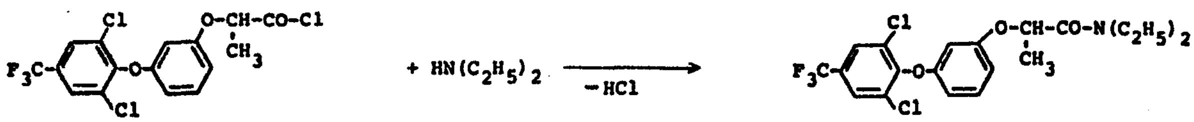


Verwendet man 2,6 - Dichlor - 4 - trifluormethyl - 3' - hydroxy - diphenyläther und α-Brompropionsäure-methylester als Ausgangsstoffe, so kann der Reaktionsablauf nach dem erfindungsgemäßen Verfahren (b) durch das folgende Formelschema wiedergegeben werden:

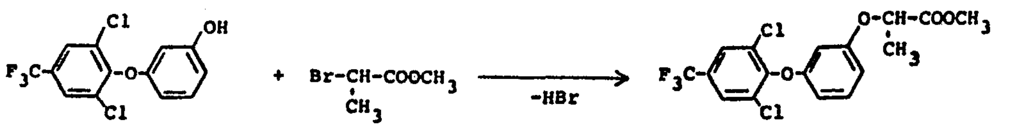


Verwendet man 3-(2,6-Dichlor-4-trifluormethyl-phenoxy)-α-phenoxy-propionsäure-methylester und Natriumhydroxid als Ausgangsstoffe, so kann der Reaktionsablauf nach dem erfindungsgemäßen Verfahren (c) durch das folgende Formelschema wiedergegeben werden:

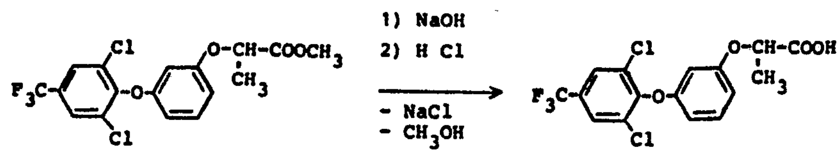


Verwendet man 3-(2,6-Dichlor-4-trifluormethyl-phenoxy)-α-phenoxy-propionsäure-methylester und n-Butanol als Ausgangsstoffe, so kann der Reaktionsablauf nach dem erfindungsgemäßen Verfahren (d) durch das folgende Formelschema wiedergegeben werden:

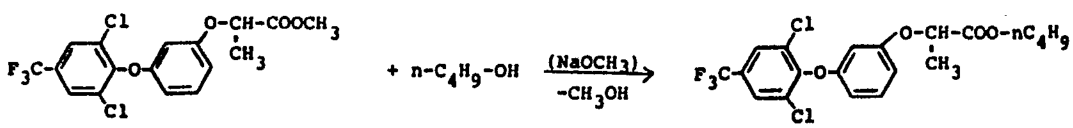


Das bei der Durchführung des erfindungsgemäßen Verfahrens (a) als Ausgangsstoff benötigte Phenoxypropionsäure-chlorid ist durch die Formel (II) genau definiert.

Die betreffende Verbindung ist bislang noch nicht bekannt. Sie läßt sich jedoch in einfacher Weise nach im Prinzip bekannten Verfahren herstellen. So erhält man das Phenoxypropionsäurechlorid der Formel (II), indem man die entsprechende Säure der Formel

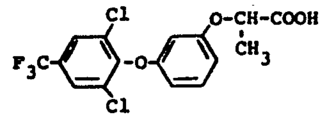

(I″)

mit Chlorierungsmitteln, wie Thionylchlorid, gegebenenfalls in Gegenwart von Verdünnungsmitteln, wie Äthylenchlorid, bei Temperaturen zwischen 0°C und 50°C, vorzugsweise zwischen 10°C und 30°C umsetzt.

Die substituierte Phenoxypropionsäure der Formel (I″) ist ebenfalls bisher noch nicht bekannt. Sie läßt sich jedoch ebenfalls in einfacher Weise nach im Prinzip bekannten Verfahren herstellen. So erhält man die substituierte Phenoxypropionsäure der Formel (I″), indem man den entsprechenden Methylester der Formel

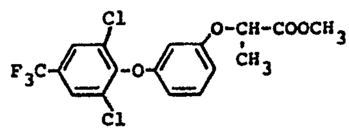 (I′)

mit starken Basen, wie z.B. Natriumhydroxid oder Kaliumhydroxid, in Gegenwart eines Verdünnungsmittels, wie Methanol oder Wasser, bei Temperaturen zwischen 20°C und 100°C, vorzugsweise zwischen 40°C und 80°C behandelt.

Der substituierte Phenoxypropionsäuremethylester der Formel (I′) ist ebenfalls bisher noch nicht bekannt. Er läßt sich jedoch ebenfalls in einfacher Weise nach im Prinzip bekannten Verfahren herstellen. So erhält man den substituierten Phenoxypropionsäuremethylester der Formel (I′), indem man den Diphenyläther der Formel

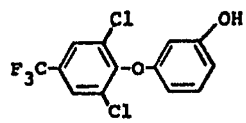 (IV)

mit $\alpha$-Brompropionsäure-methylester der Formel

$$Br-\underset{\displaystyle CH_3}{\underset{|}{CH}}-COOCH_3 \qquad (V)$$

in Gegenwart eines Säurebindemittels, wie Natriummethylat, Natriumäthylat oder Kaliumcarbonat, in Gegenwart eines polaren Verdünnungsmittels, wie Methanol oder Acetonitril, bei Temperaturen zwischen 20°C und 100°C, vorzugsweise 30°C und 80°C umsetzt.

Der Diphenyläther der Formel (IV) ist bisher noch nicht bekannt. Er läßt sich jedoch in einfacher Weise nach im Prinzip bekannten Verfahren herstellen. So erhält man den Diphenyläther der Formel (IV), indem man halogenierte Trifluormethyl-benzole der Formel

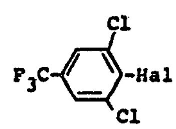 (VII)

in welcher
Hal für Chlor oder Brom steht,
mit Resorcin der Formel

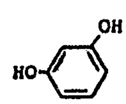 (VIII)

in Gegenwart eines Säurebindemittels sowie in Gegenwart eines Verdünnungsmittels bei Temperaturen zwischen 20°C und 200°C, vorzugsweise zwischen 50°C und 150°C umsetzt.

Als Säurebindemittel kommen hierbei alle üblichen Basen in Frage. Hierzu gehören vorzugsweise Alkalihydroxide, wie Natriumhydroxid und Kaliumhydroxid und Erdalkalihydroxide, wie Calciumhydroxid.

Als Verdünnungsmittels kommen alle üblichen inerten aprotischen polaren Lösungsmittel in Frage. Hierzu gehören vorzugsweise Amide, wie Hexamethylphosphorsäuretriamid, Dimethylformamid oder Dimethylacetamid, ferner Sulfoxide, wie Dimethylsulfoxid, weiterhin Ketone, wie Methyl-äthylketon, außerdem Nitrile, wie Acetonitril und Sulfolan.

Die halogenierten Trifluormethyl-benzole der Formel (VII) sind bekannt.

Die bei der Durchführung des erfindungsgemäßen Verfahrens (a) weiterhin als Ausgangsstoffe verwendbaren Verbindungen sind durch die Formel (III) allgemein definiert. In der Formel (III) steht R vorzugsweise für diejenigen Reste, die bereits im Zusammenhang mit der Beschreibung der erfindungsgemäßen Stoffe der Formel (I) vorzugsweise für R genannt wurden.

Die Verbindungen der Formel (III) sind bekannt. Als Beispiele seien im einzelnen genannt:

Wasser
Methanol
Äthanol
Propanol
Isopropanol
n-Butanol
tert.-Butanol
2-Methoxy-äthanol
2-Äthoxy-äthanol
2-Methylthio-äthanol
Äthoxycarbonyl-methanol
2-Äthoxycarbonyl-äthanol
2-Phenylmethylaminocarbonyl-äthanol
2-Chlor-äthanol
Ammoniak
Hydrazin
Methylamin
Äthylamin
n-Propylamin
Isopropylamin
2-Methoxy-äthylamin
2-Dimethylamino-äthylamin
Dimethylamin
Diäthylamin
Di-n-propylamin
Buten-2-yl-amin
Anilin
Phenyl-methyl-amin
4-Methyl-anilin
4-Methoxy-anilin
4-Äthoxycarbonyl-anilin
4-Chloranilin
4-Nitro-anilin
Piperidin
2-Methylpiperidin
Pyrrolidin
Piperazin
Pyrazolidin
Morpholin
2-Benzothiazolyl-methyl-amin

Als Verdünnungsmittel kommen bei der Durchführung des erfindungsgemäßen Verfahrens (a) alle inerten organischen Lösungsmittel in Betracht. Hierzu gehören vorzugsweise Ketone, wie Diäthylketon und Methyl-isobutyl-keton, ferner Nitrile, wie Acetonitril und Propionitril weiterhin Äther, wie Tetrahydrofuran und Dioxan, darüber hinaus aliphatische und aromatische Kohlenwasserstoffe, wie Petroläther, Benzol, Toluol und Xylol, außerdem halogenierte Kohlenwasserstoffe, wie Methylenchlorid, Tetrachlorkohlenstoff, Chloroform und Chlorbenzol, außerdem Ester wie Essigester, sowie Formamide, wie Dimethylformamid.

In manchen Fällen, in denen es sich bei der Verbindung der Formel (III) um eine flüssige Komponente handelt, erübrigt sich der Zusatz eines Verdünnungsmittels. Es kann jedoch auch in diesen Fällen ein Verdünnungsmittel hinzugefügt werden.

Als Säurebindemittel können bei der Durchführung des erfindungsgemäßen Verfahrens (a) alle üblicherweise verwendbaren anorganischen und organischen Säureakzeptoren eingesetzt werden. Hierzu gehören vorzugsweise Alkalicarbonate, beispielsweise Natriumcarbonat, Kaliumcarbonat und Natriumhydrogencarbonat, ferner niedere tertiäre Alkylamine, Aralkylamine, aromatische Amine oder Cycloalkylamine, wie z.B. Triäthylamin, Dimethylbenzylamin, Pyridin und Diazabicyclooctan.

Handelt es sich bei der Verbindungen der Formel (III) um ein Amin, so kann auch dieses im Überschuß eingesetzte Amin als Säurebindemittel fungieren.

Die Reaktionstemperaturen können bei dem erfindungsgemäßen Verfahren (a) in einem größeren Bereich variiert werden. Im allgemeinen arbeitet man bei Temperaturen zwischen 20°C und 100°C, vorzugsweise zwischen 30°C und 80°C.

Bei der Durchführung des erfindungsgemäßen Verfahrens (a) setzt man auf 1 Mol an Phenoxypropionsäurechlorid der Formel (II) vorzugsweise 1 bis 2 Mol oder auch einen größeren Überschuß an

einer Verbindung der Formel (III) sowie gegebenenfalls 1 bis 2 Mol an Säurebindemittel ein. — Die Isolierung der erfindungsgemäßen Stoffe der Formel (I) erfolgt nach üblichen Methoden. Im allgemeinen verfährt man in der Weise, daß man das Reaktionsgemisch nach beendeter Umsetzung entweder direkt oder nach vorherigem Einengen unter vermindertem Druck mit einem organischen Lösungsmittel, wie Toluol oder Methylenchlorid, versetzt, die organische Phase dann abtrennt, wäscht und nach dem Trocknen einengt.

Der bei der Durchführung des erfindungsgemäßen Verfahrens (b) als Ausgangsstoff verwendbare Diphenyläther ist durch die Formel (IV) genau definiert.

Der Diphenyläther der Formel (IV) ist bisher noch nicht bekannt. Man erhält diesen Stoff z.B. nach der Methode, die bereits im Zusammenhang mit der Herstellung des Ausgangsproduktes für das Verfahren (a) im einzelnen beschrieben wurde.

Der bei dem erfindungsgemäßen Verfahren (b) weiterhin als Ausgangsstoff benötigte $\alpha$-Brompropionsäure-methylester der Formel (V) ist bekannt.

Die bei der Durchführung des erfindungsgemäßen Verfahrens (b) einzuhaltenden Reaktionsbedingungen wurden ebenso wie die bei der Umsetzung zu verwendenden Säurebindemittel und Verdünnungsmittel bereits im Zusammenhang mit der Herstellung des Ausgangsstoffes für das erfindungsgemäße Verfahren (a) im einzelnen beschrieben.

Bei der Durchführung des erfindungsgemäßen Verfahrens (b) setzt man auf 1 Mol Diphenyläther der Formel (IV) vorzugsweise 1 bis 2 Mol $\alpha$-Brompropionsäure-methylester der Formel (V) sowie 1 bis 2 Mol an Säurebindemittel ein. Die Isolierung des Reaktionsproduktes erfolgt im Falle des erfindungsgemäßen Verfahrens (b) nach üblichen Methoden. Im allgemeinen verfährt man in der Weise, daß man nach beendeter Umsetzung das Reaktionsgemisch einengt, den verbleibenden Rückstand in einem organischen Lösungsmittel, wie Methylenchlorid, aufnimmt, die Lösung filtriert, wäscht und nach dem Trocknen unter vermindertem Druck einengt.

Der bei der Durchführung des erfindungsgemäßen Verfahrens (c) als Ausgangsstoff benötigte Phenoxypropionsäure-methylester ist durch die Formel (I') genau definiert.

Der Phenoxypropionsäure-methylester der Formel (I') ist bislang noch nicht bekannt. Er läßt sich jedoch in einfacher Weise nach im Prinzip bekannten Verfahren herstellen. Man erhält diesen Stoff z.B. nach der Methode die bereits im Zusammenhang mit der Herstellung der Ausgangsstoffe für das Verfahren (a) im einzelnen beschrieben wurde.

Die bei der Durchführung des erfindungsgemäßen Verfahrens (c) einzuhaltenden Reaktionsbedingungen wurden ebenso wie die bei der Umsetzung zu verwendenden Basen und Verdüngsmittel bereits im Zusammenhang mit der Herstellung der Ausgangsstoffe für das erfindungsgemäße Verfahren (a) im einzelnen beschrieben.

Bei der Durchführung des erfindungsgemäßen Verfahrens (c) setzt man auf 1 Mol an Phenoxypropionsäure-methylester der Formel (I') vorzugsweise 1 bis 2 Mol an Base ein. Die Isolierung des Reaktionsproduktes erfolgt im Falle des erfindungsgemäßen Verfahrens (c) nach üblichen Methoden. Im allgemeinen verfährt man in der Weise, daß man nach beendeter Umsetztung das Reaktionsgemisch in Wasser gießt, die Lösung filtriert, dann ansäuert und die dabei in fester Form anfallende Säure absaugt.

Bei der Durchführung des erfindungsgemäßen Verfahrens (d) dient derjenige Phenoxypropionsäure-methylester als Ausgangsstoff, der bereits im Zusammenhang mit der Beschreibung des erfindungsgemäßen Verfahrens (c) erwähnt wurde.

Die bei dem erfindungsgemäßen Verfahren (d) weiterhin als Ausgangsstoffe zu verwendenden Alkohole sind durch die Formel (VI) allgemein definiert. In der Formel (VI) steht $R^1$ vorzugsweise für geradkettiges oder verzweigtes Alkyl mit 2 bis 4 Kohlenstoffatomen.

Als Beispiele für Verbindungen der Formel (VI) seien im einzelnen genannt:

Äthanol
n-Propanol
Isopropanol
i-Butanol
n-Butanol
sec.-Butanol
tert.-Butanol

Die Alkohole der Formel (VI) sind bekannt.

Bei dem erfindungsgemäßen Verfahren (d) können die im Überschuß eingesetzten Reaktionskomponenten der Formel (VI) gleichzeitig als Verdünnungsmittel fungieren. Es ist jedoch auch möglich, inerte organische Lösungsmittel als Verdünnungsmittels zu verwenden.

Die Umsetzung nach dem erfindungsgemäßen Verfahren (d) wird vorzugsweise in Gegenwart eines Alkoholates, wie z.B. Natriummethylat, durchgeführt.

Die Reaktionstemperaturen können bei dem erfindungsgemäßen Verfahren (d) innerhalb eines größeren Bereiches variiert werden. Im allgemeinen arbeitet man bei Tempraturen zwischen 20°C und 200°C, vorzugsweise zwischen 60°C und 120°C.

Bei der Durchführung des erfindungsgemäßen Verfahrens (d) setzt man auf 1 Mol an Phenoxypropionsäure-methylester der Formel (I') einen Überschuß an Alkohol der Formel (VI) sowie zweckmäßigerweise eine katalytische Menge an Alkoholat ein. — Die Isolierung der Reaktionsprodukte erfolgt nach üblichen Methoden. Im allgemeinen verfährt man in der Weise, daß man das Reaktionsgemisch nach beendeter Umsetzung einengt, den verbleibenden Rückstand mit einem organischen Lösungsmittel verrührt, die entstehende Lösung filtriert, wäscht und nach dem Trocknen einengt.

Die erfindungsgemäßen Wirkstoffe beeinflussen das Pflanzenwachstum und können deshalb als Defoliants, Desiccants, Krautabtötungsmittel, Reimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B bei den folgenden Pflanzen verwendet werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Impomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dicotyle Kulturen der Gattungen:* Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Spenoclea, Dactyloctenium, Agrostis, Alopecurus, Apera.

*Monokotyle Kulturen der Gattungen:* Oryza, Zea, Triticum, Rordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Besonders geeignet sind die erfindungsgemäßen Wirkstoffe zur Bekämpfung von Cyperus. Darüber hinaus können sie sehr gut zur Bekämpfung von Unkräutern in Getreide, Mais und Reis verwendet werden.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden und Akariziden.

Die Wirkstoffe können als solche, in Form ihrer Formulierungen oder der daraus bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Spritzen, Sprühen, Stäuben, Streuen und Gießen.

Die Anwendung ist sowohl nach dem post-emergence-Verfahren als auch nach dem pre-emergence-Verfahren möglich. Bei der pre-emergence-Anwendung zeigt sich eine bessere Selektivität als bei der post-emergence-Anwendung.

Die eingesetzte Wirkstoffmenge kann in größeren Bereichen schwanken. Sie hängt im wesentlichen ab von der Art des gewünschten Effektes. Im allgemeinen liegen der Aufwandmengen zwischen 0,1 und 25 kg/ha,vorzugsweise zwischen 0,25 und 10 kg/ha.

Die erfindungsgemäßen Verbindungen besitzen nicht nur herbizide Eigenschaften, sondern besitzen außerdem auch eine fungizide Wirksamkeit.

Die gute herbizide Wirksamkeit der erfindungsgemäßen Wirkstoffe geht aus den nachfolgenden Beispielen hervor:

## Beispiel A

Pre-emergence-Test

Lösungsmittel: 5 Gewichtsteile Aceton

Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Wirkstoffe, Aufwandmengen und Resultate gehen aus der nachfolgenden Tabelle hervor:

TABELLE A

Pre-emergence-Test

| Wirkstoff | Wirkstoff-aufwand kg/ha | Sinapis | Matricaria | Galinsoga | Stellaria | Chenopodium | Cyperus | Weizen | Hafer | Baumwolle | Mais |
|---|---|---|---|---|---|---|---|---|---|---|---|
| (1) *) | 5 | 90 | 100 | 100 | 100 | 90 | 100 | 20 | 40 | 60 | 20 |
| (2) | 5 | 90 | 100 | 100 | 100 | 100 | 100 | 0 | 0 | 60 | 0 |
| (3) | 5 | 90 | 100 | 100 | 100 | 100 | 100 | 0 | 0 | 40 | 0 |

*) vgl. Wirkstoffliste auf Seite 17.

Beispiel B

Post-emergence-Test

Lösungsmittel: 5 Gewichtsteile Aceton

Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der wirkstoffzubereitung spritzt man Testpflanzen, welche eine Höhe von 5—15 cm haben so, daß die in der Tabelle angegebenen Wirkstoffmengen pro Flächeneinheit ausgebracht werden. Die Konzentration der Spritzbrühe wird so gewählt, daß in 2000 1 Wasser/ha die in der Tabelle angegebenen Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Wirkstoffe, Aufwandmengen und Resultate gehen aus der nachfolgenden Tabelle hervor:

TABELLE B

Post-emergence-Test

| Wirkstoff | Wirkstoff-aufwand kg/ha | Chenopodium | Sinapis | Galinsoga | Stellaria | Matricaria | Cyperus | Hafer | Weizen | Mais | Reis |
|---|---|---|---|---|---|---|---|---|---|---|---|
| (1) *) | 1 | 100 | 100 | 100 | 100 | 100 | 100 | 40 | 40 | 80 | 10 |
| (2) | 1 | 90 | 100 | 100 | 90 | 80 | 100 | 40 | 40 | 80 | 10 |
| (3) | 1 | 60 | 100 | 40 | 60 | 60 | 100 | 0 | 40 | 80 | 20 |

*) vgl. Wirkstoffliste auf Seite 17.

Wirkstoff-Liste

(1) = 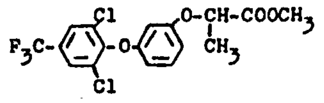

(2) = 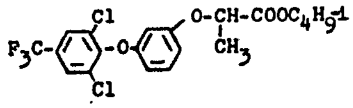

(3) = 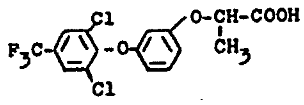

## Herstellungsbeispiele

### Beispiel 1

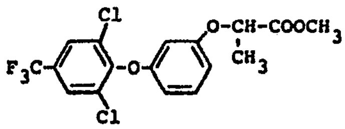

In eine Lösung von 97 g (0,3 Mol) 2,6-Dichlor-4-trifluormethyl-3'-hydroxy-diphenyläther und 17,8 g Natriummethylat in 400 ml Methanol werden bei einer Temperatur von 45 bis 50°C innerhalb von 2 Stunden 55,1 g $\alpha$-Brom-propionsäure-methylester eingetropft. Man läßt 3 Stunden bei 45—50°C nachrühren und arbeitet dann auf, indem man das Reaktionsgemisch einengt, den verbleibenden Rest in 1 l Methylenchlorid aufnimmt, die Lösung filtriert, dann nacheinander mit verdünnter wäßriger Natronlauge und Wasser wäscht und nach dem Trocknen einengt. Man erhält auf diese Weise 59,0 g (65,4% der Theorie) an 3-(2,6-Dichlor-4-trifluormethyl-phenoxy)-$\alpha$-phenoxy-propionsäure-methylester in Form eines bräunlichen Öles, ($n_D^{20}$ = 1,5206).

Analyse:

Summenformel: $C_{17}H_{13}Cl_2F_3O_4$

Berechnet: 49,9% C; 3,2% H; 17,4% Cl

Gefunden: 49.6% C; 3,6% H; 16,8% Cl

Herstellung des als Ausgangsprodukt benötigten 2,6-Dichlor-4-trifluormethyl-3'-hydroxy-diphenyl-äthers der Formel

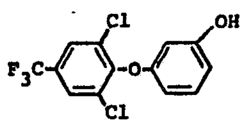

In ein Gemisch aus 330 g (3 Mol) Resorcin und 111 g (1,5 Mol) Calciumhydroxid in 2 l Dimethylsulfoxid werden bei 120—130°C innerhalb von 7 Stunden 358 g (1,5 Mol) 3,4,5-Trichlor-benzotrichlorid eingetropft. Anschließend wird die Reaktionsmischung noch 8 Stunden bei 130°C nachgerührt. Nach dem Abkühlen auf Raumtemperatur wird das Reaktionsgemisch in 7 l Wasser gegossen, wobei sich das Reaktionsprodukt ölig abscheidet. Man extrahiert mit 3 l Toluol, trennt die organische Phase ab und engt nach dem Trocknen ein. Der verbleibende Rückstand wird destilliert. Man erhält auf diese Weise 350 g (73% der Theorie) an 2,6-Dichlor-4-trifluormethyl-3'-hydroxy-diphenyläther in Form einer Festsubstanz von Schmelzpunkt 64—65°C.

Sdp. 123—130°C/0,15 Torr

Analyse:

Summenformel: $C_{13}H_7Cl_2\ F_3O_2$

Berechnet: 48,3% C; 2,2% H; 22,0% Cl

Gefunden: 48,3% C; 2,3% H; 21,8% Cl

Beispiel 2

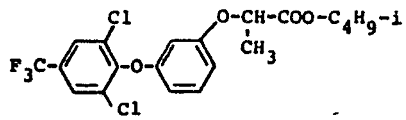

Ein Gemisch aus 20,5 g (0,05 Mol) 3-(2,6-Dichlor-4-trifluormethyl-phenoxy)-$\alpha$-phenoxy-propionsäure-methylester, 0,1 g Natriummethylat und 150 ml iso-Butanol wird 9 Stunden unter Rückfluß gekocht. Danach wird das überschüssige i-Butanol bei Normaldruck abdestilliert. Anschließend arbeitet man auf, indem man das Reaktionsgemisch weiter einengt, den verbleibenden Rückstand mit 500 ml Methylenchlorid verrührt, danach zweimal mit Wasser wäscht und nach dem Trocknen einengt. Man erhält auf diese Weise 17,5 g (77,6% der Theorie) an 3-(2,6-Dichlor-4-trifluormethyl-phenoxy)-$\alpha$-phenoxy-propionsäure-iso-butylester in Form eines gelblichen Öles.

Analyse:

Summenformel: $C_{20}H_{19}Cl_2F_3O_4$

Berechnet: 53,2% C; 4,2% H; 15,7% Cl

Gefunden: 53,1% C; 4,5% H; 15,4% Cl

Beispiel 3

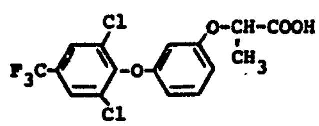

Zu 27,5 g (0,067 Mol) 3-(2,6-Dichlor-4-trifluormethyl-phenoxy)-$\alpha$-phenoxy-propionsäure-methylester und 70 ml Methanol gibt man 30 ml Wasser und 10 ml konzentrierte Natronlauge und läßt 5 Stunden bei 50—60°C nachrühren. Anschließend arbeitet man auf, indem man das Reaktionsgemisch in 1 l Wasser gießt, die Lösung filtriert, ansäuert und das kristallin anfallende Produkt absaugt. Man erhält auf diese Weise 21,7 g (82,0% der Theorie) an 3-(2,6-Dichlor-4-trifluormethyl-phenoxy)-$\alpha$-phenoxy-propionsäure in Form einer elfenbeinfarbenen Festsubstanz vom Schmelzpunkt 151°C.

Beispiel 4

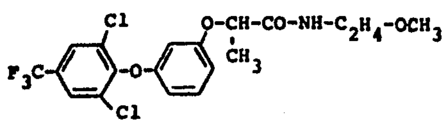

In eine Lösung von 11,3 g (0,05 Mol) (2-Amino-äthyl)-methyläther in 10 ml Pyridin tropft man bei 0—5°C eine Lösung von 20,7 g 3-(2,6-Dichlor-4-trifluormethyl-phenoxy)-$\alpha$-phenoxy-propionsäure-chlorid in 30 ml Toluol zu. Man läßt über Nacht bei Raumtemperatur rühren und arbeitet dann auf, indem man das Reaktionsgemisch mit 350 ml Toluol versetzt, dann nacheinander mit verdünnter wäßriger Natronlauge und Wasser bis zur neutralen Reaktion wäscht und nach dem Trocknen einengt. Man erhält auf diese Weise 19,7 g (87,2% der Theorie) an 3-(2,6-Dichlor-4-trifluormethyl-phenoxy)-$\alpha$-phenoxy-propionsäure-(2-methoxy)-äthylamid in Form einer farblosen Festsubstanz vom Schmelzpunkt 90—100°C.

Herstellung des als Ausgangsprodukt benötigten 3-(2,6-Dichlor-4-trifluormethyl-phenoxy)-$\alpha$-phenoxy-propionsäure-chlorids der Formel

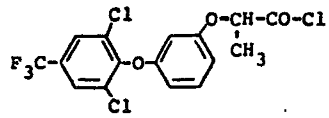

In eine Lösung von 98,2 g (0,249 Mol) 3-(2,6-Dichlor-4-trifluormethyl-phenoxy)-α-phenoxy-propionsäure und 3 ml Dimethylformamid in 200 ml Äthylenchlorid werden bei Raumtemperatur 35,5 g (0,298 Mol) Thionylchlorid eingetropft. Danach wird das Reaktionsgemisch langsam auf Siedetemperatur erhitzt und 4 Stunden unter Rückfluß erhitzt. Anschließend arbeitet man auf, indem mar das Reaktionsgemisch filtriert und einengt. Man erhält auf diese Weise 88,5 g and 3-(2,6-Dichlor-4-trifluormethyl-phenoxy)-α-phenoxy-propionsäure-chlorid in Form eines orangefarbenen Öles.

Analyse:

Berechnet: 46,4% C; 2,4% H; 25,8% Cl

Gefunden: 45,7% C; 3,0% H; 26,0% Cl

Nach einem oder mehren der vorstehend beschriebenen Verfahren werden die in der nachfolgenden Tabelle formelmäßig aufgeführten Stoffe hergestellt.

TABELLE 1

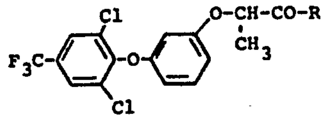

| Beisp.-Nr. | R | Schmelzpunkt [°C] bzw. Brechungsindex [$n_D^{20}$] |
|---|---|---|
| 5 | 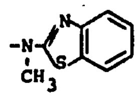 | 136 |
| 6 | 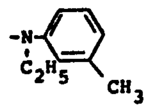 | 77 |
| 7 | 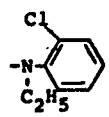 | 1,5476 |
| 8 | 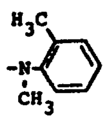 | 1,5585 |
| 9 | $-O-C_2H_4-S-C_2H_5$ | 1,5342 |
| 10 | 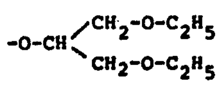 | 1,5062 |
| 11 | 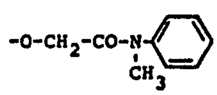 | 67–68 |
| 12 | $-NH-CH_2-CH=CH_2$ | 120 |
| 13 | 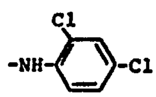 | 100 |
| 14 | 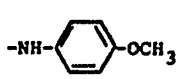 | 149 |

TABELLE 1 (Fortsetzung)

| Beisp.-Nr. | R | Schmelzpunkt [°C] bzw. Brechungsindex [$n_D^{20}$] |
|---|---|---|
| 15 | 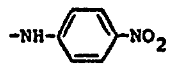 | 63–64 |
| 16 | 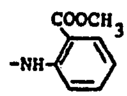 | 116 |
| 17 | 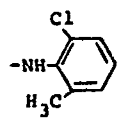 | 161 |
| 18 | 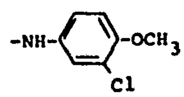 | 188 |
| 19 | $-NH-C_2H_4-N(C_2H_5)_2$ | 39–40 |
| 20 | 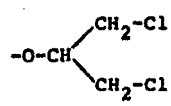 | 95 |
| 21 | $-NH_2$ | 114–115 |
| 22 | $-N(CH_3)_2$ | 89–90 |
| 23 | 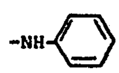 | 105–106 |
| 24 | 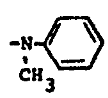 | 82–84 |
| 25 | 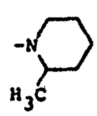 | 1,5393 |
| 26 | $-NH-NH_2$ | 95 |
| 27 | $-O-CH_2-COOC_2H_5$ | 1,5167 |

**Patentansprüche**

1. Phenoxypropionsäure-Derivate der Formel

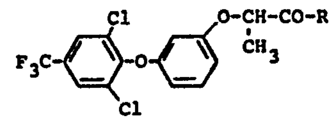

(I)

in welcher

R für Hydroxy, gegebenenfalls durch Alkoxy, Alkylthio, Carbalkoxy, Phenylmethylaminocarbonyl und/oder Halogen substituiertes Alkoxy, Amino, Hydrazino, gegebenenfalls durch Alkoxy oder Dialkylamino substituiertes Alkylamino, Dialkylamino, Alkenylamino, Arylamino oder N-Aryl-N-alkyl-amino, wobei der Arylrest jeweils durch Alkyl, Alkoxy, Carbalkoxy, Halogen und/oder Nitro substituiert sein kann, ferner für einen gegebenenfalls durch Alkyl substituierten, über Stickstoff gebundenen gesättigten heterocyclischen Ring mit 5 oder 6 Ringgliedern, oder für den Rest der Formel

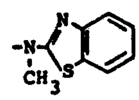

steht.

2. Verfahren zur Herstellung von Phenoxypropionsäure-Derivaten, dadurch gekennzeichnet, daß man

a) das Phenoxypropionsäure-chloride der Formel

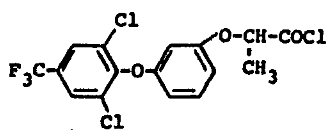

(II)

mit Verbindungen der Formel

HR (III)

in welcher

R die oben angegebene Bedeutung hat,

gegebenenfalls in Gegenwart eines Säureakzeptors sowie gegebenenfalls in Gegenwart eines Verdünnungsmittels umsetzt,

oder

b) zur Herstellung derjenigen Verbindung der Formel (I), in der R für Methoxy steht, den Diphenyläther der Formel

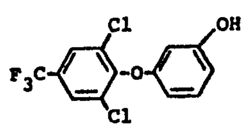

(IV)

mit $\alpha$-Brompropionsäure-methylester der Formel

$$\underset{\displaystyle CH_3}{\overset{}{Br—CH—COOCH_3}} \quad (V)$$

in Gegenwart eines Säureakzeptos sowie in Gegenwart eines Verdünnungsmittels umsetzt.

oder

c) zur Herstellung derjenigen Verbindung der Formel (I), in der R für Hydroxyl steht, die Verbindungen der Formel

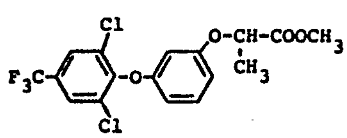

(I')

mit Basen in Gegenwart eines Verdünnungsmittels behandelt und anschließend ansäuert. oder

d) zur Herstellung derjenigen Verbindungen der Formel (I), in denen R für Alkoxy mit mehr als einem Kohlenstoffatomen steht, Verbindungen der Formel

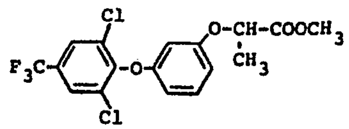

(I')

mit Alkoholen der Formel

$$HOR^1 \qquad (VI)$$

in welcher

$R^1$ für Alkyl mit mehr als einem Kohlenstoffatomen steht,

gegebenenfalls in Gegenwart eines Alkoholates sowie gegebenenfalls in Gegenwart eines Verdünnungsmittels umsetzt.

3. Herbizide Mittel, gekennzeichnet durch einen Gehalt an mindestens einem Phenoxypropionsäure-Derivat gemäß Anspruch 1.

4. Verwendung von Phenoxypropionsäure-Derivaten gemäß Anspruch 1 zur Bekämpfung von Unkräutern.

5. Verfahren zur Herstellung herbizider Mittel, dadurch gekennzeichnet, daß man Phenoxypropionsäure-Derivate gemäß Anspruch 1 mit Streckmitteln und/oder oberflächenaktiven Mitteln vermischt.

6. 3-(2,6-Dichlor-4-trifluormethyl-phenoxy)-$\alpha$-phenoxy-propionsäure-chlorid.

**Revendications**

1. Dérivés d'acide phénoxypropionique de formule

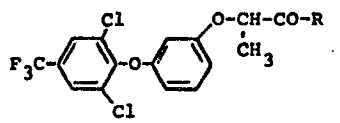

(I)

dans laquelle

R représente un hydroxy, un alcoxy éventuellement substitué par un alcoxy, alcoylthio, carbalcoxy, phenylméthylaminocarbonyle et/ou de l'halogène, un amino, un hydrazino, un alcoylamino éventuellement substitué par un alcoxy ou dialcoylamino, un dialcoylamino, un alcénylamino, un arylamino ou un N-aryl-N-alcoyl-amino, le radical aryle pouvant être chaque fois substitué par un alcoyle, alcoxy, carbalcoxy, halogène et/ou nitro, en outre un noyau hétérocyclique saturé à 5 ou 6 chaînons nucléaires, lié par de l'azote et éventuellement substitué par un alcoyle, ou le radical de formule:

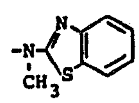

2. Procédé de préparation de dérivés d'acide phénoxypropionique, caractérisé en ce que

a) on fait réagir le chlorure de phénoxypropionyle de formule:

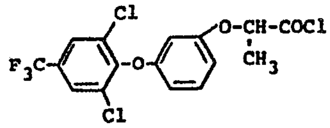

(II)

avec le composé de formule

$$HR \qquad (III)$$

dans laquelle

R a la signification indiquée plus haut, éventuellement en présence d'un accepteur d'acide de même qu'éventuellement en présence d'un diluant, ou

b) pour la préparation d'un composé de formule (I) dans laquelle R représente un méthoxy, on fait réagir le diphényl-éther de formule:

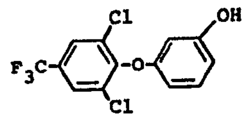

(IV)

avec de l'$\alpha$-bromopropionate de méthyle de formule:

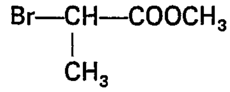

(IV)

en présence d'un accepteur d'acide de même qu'en présence d'un diluant,
ou

c) pour la préparation d'un composé de formule (I) dans laquelle R représente un hydroxyle, on traite le composé de formule:

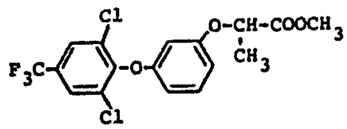

(I')

avec des bases en présence d'un diluant et on acidifie ensuite,
ou

d) pour la préparation des composés de formule (I) dans laquelle R représente un alcoxy ayant plus d'un atome de carbone, on fait réagir des composés de formule:

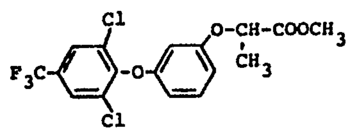

(I')

avec des alcools de formule:

$$HOR^1 \qquad (VI)$$

dans laquelle
$R^1$ représente un alcoyle ayant plus d'un atome de carbone,
éventuellement en présence d'un alcoolate de même éventuellement qu'en présence d'un diluant.

3. Agents herbicides, caractérisés par une teneur en au moins un dérivé d'acide phénoxypropionique selon la revendication 1.

4. Utilisation de dérivés d'acide phénoxypropionique selon la revendication 1 pour combattire les plantes adventices.

5. Procédé de fabrication d'agents herbicides, caractérisé en ce qu'on mélange des dérivés d'acide phénoxypropionique selon la revendication 1 avec des diluants et/ou de agents tensioactifs.

6. Chlorure de 3-(2,6-dichloro-4-trifluorméthyl-phénoxy)-$\alpha$-phénoxy-propionyle.

**Claims**

1. Phenoxypropionic acid derivatives of the formula

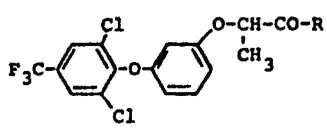

(I)

in which
R represents hydroxyl, alkoxy which is optionally substituted by alkoxy, alkylthio, carbalkoxy, phenylmethylaminocarbonyl and/or halogen; amino, hydrazino, alkylamino which is optionally substituted by alkoxy or dialkylamino, dialkylamino, alkenylamino, arylamino or N-aryl-N-alkylamino, it being possible for the aryl radical in each case to be substituted by alkyl, alkoxy, carbalkoxy, halogen and/or nitro, and furthermore represents a saturated heterocyclic ring with 5 or 6 ring members which is optionally substituted by alkyl and is bonded via nitrogen, or represents the radical of the formula

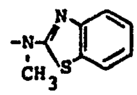

2. Process for the preparation of phenoxypropionic acid derivatives, characterised in that a) phenoxypropionic acid chlorides of the formula

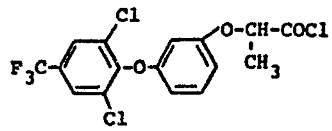

(II)

are reacted with compounds of the formula

HR (III)

in which

R has the meaning indicated above, optionally in the presence of an acid acceptor and optionally in the presence of a diluent, or

b) in order to prepare the compound of the formula (I) in which R represents methoxy, the diphenyl ether of the formula

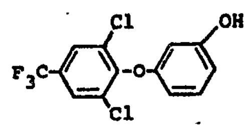

(IV)

is reacted with $\alpha$-bromopropionic acid methyl ester of the formula

$$Br-\underset{\displaystyle CH_3}{\underset{|}{CH}}-COOCH_3 \qquad (V)$$

in the presence of an acid acceptor and in the presence of a diluent, or

c) in order to prepare the compound of the formula (I) in which R represents hydroxyl, compounds of the formula

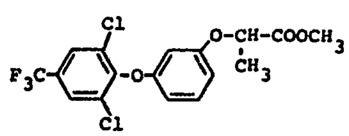

(I')

are treated with bases in the presence of a diluent and then acidified or

d) in order to prepare those compounds of the formula (I) in which R represents alkoxy with more than one carbon atom, compounds of the formula

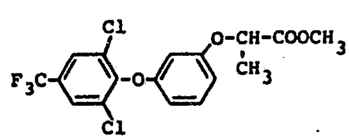

(I')

are reacted with alcohols of the formula

$HOR^1$ (VI)

in which

$R^1$ represents alkyl with more than one carbon atom, optionally in the presence of an alcoholate and optionally in the presence of a diluent.

3. Herbicidal agents, characterised in that they contain at least one phenoxypropionic acid derivative according to Claim 1.

4. Use of phenoxypropionic acid derivatives according to Claim 1 for combating weeds.

5. Process for the preparation of herbicidal agents, characterised in that phenoxypropionic acid derivatives according to Claim 1 are mixed with extenders and/or surface-active agents.

6. 3-(2,6-Dichloro-4-trifluoromethyl-phenoxy)-$\alpha$-phenoxypropionic acid chloride.